# EUROPÄISCHE PATENTSCHRIFT

(11) **EP 1 985 245 B1**
(45) Veröffentlichungstag und Bekanntmachung des Hinweises auf die Patenterteilung: **06.01.2010**
(21) Anmeldenummer: 07008200.3
(22) Anmeldetag: 23.04.2007
(51) Int. Cl.: A61B 17/16, A61C 1/12

(54) **Medizinisches Handgriffelement**
Medical handle element
Elément de poignée médical

(43) Veröffentlichungstag der Anmeldung: 29.10.2008
(73) Patentinhaber: W & H Dentalwerk Bürmoos GmbH, 5111 Bürmoos (AT)
(72) Erfinder: Ploy, Gernot, 5111 Bürmoos (AT)

(56) Entgegenhaltungen:
- EP-A1- 1 157 671
- DE-B- 1 061 033
- US-A- 5 340 311

## Beschreibung

Die vorliegende Erfindung betrifft ein medizinisches, insbesondere dentales, Handgriffelement nach dem Oberbegriff des Anspruchs 1.

Aus dem Dokument US 4,341,520 ist ein Handgriffelement mit einem drehbar in Wälzlagern gelagerten Drehteil, das als Laufrad mit einer Werkzeughalterung ausgebildet ist, bekannt. Die Wälzlager bzw. die äußeren Laufringe der beiden Wälzlager sind durch O-Ringe aus Elastomer im Kopfteil des Handgriffelements vorgespannt und vor Verdrehung relativ zum Kopfteil gesichert. Dieser Aufbau ist seit langem bekannt und weit verbreitet.

Ein Nachteil der Verwendung von elastomeren O-Ringen zur Vorspannung und Verdrehsicherung von Wälzlagern ist ihr Verschleiß bei wiederholter Reinigung oder Sterilisation. Die O-Ringe sind dabei unterschiedlichen Einflüssen wie Druckschwankungen und Temperaturschwankungen mit Temperaturen von über 120°C, oftmals über 130°C, Wasserdampf oder aggressiven chemischen Substanzen wie Reinigungs- oder Desinfektionsmitteln ausgesetzt. Durch diese Einflüsse quillt der elastomere O-Ring auf und verliert seine Elastizität, so dass er seine Funktionen nicht mehr zuverlässig ausüben kann.

Ein weiteres derartiges Handgriffelement mit einem elastischen O-Ring zur Lagerung der äußeren Laufringe der Kugellager ist in dem Dokument EP 1 157 671 A1 beschrieben.

Aus dem Dokument DE 10 61 033 ist ein Handgriffelement mit einer Sicherungsschraube zur Lagensicherung der das Laufrad und die Werkzeugaufnahme lagernden Kugellager bekannt. Die Sicherungsschraube fixiert die beiden Außenringe der Kugellager mit Hilfe eines Zwischenrings, wobei die Sicherungsschraube mit dem Gehäuse des Handgriffelements verschraubbar ist.

Das Dokument US 5,340,311 offenbart ein Handgriffelement mit einer Schraubenmutter aus Kunststoff zur Verdrehsicherung der das Laufrad und die Werkzeugaufnahme lagernden Kugellager.

Der vorliegenden Erfindung liegt daher die Aufgabe zugrunde ein medizinisches, insbesondere dentales, Handgriffelement ohne die oben genannten Nachteile zu schaffen, wobei das Handgriffelement insbesondere eine gegenüber Einflüssen von Sterilisations- oder Reinigungsprozessen weniger empfindliche Verdrehsicherung für die äußeren Laufringe der Wälzlager aufweisen soll.

Diese Aufgabe wird gemäß der vorliegenden Erfindung durch ein medizinisches, insbesondere dentales, Handgriffelement mit den Merkmalen des Anspruchs 1 und ein Verfahren zu dessen Herstellung mit den Merkmalen des Anspruchs 10 gelöst. Besonders vorteilhafte Ausführungsformen sind in den Unteransprüchen angeführt.

Das erfindungsgemäße medizinische, insbesondere dentale, Handgriffelement umfasst ein drehbar angeordnetes Drehteil und zumindest ein Wälzlager, insbesondere ein Kugellager, zur Lagerung des drehbaren angeordneten Drehteils. Das Wälzlager ist an einem Lagersitz angeordnet und weist zumindest ein mit dem Drehteil mitrotierbares Wälzlagerteil und zumindest einen relativ zum Drehteil feststehendes Wälzlagerteil auf. Die beiden Wälzlagerteile sind bevorzugt durch zwei Laufbahnen gebildet, in denen mehrere Wälzkörper geführt sind. Das Handgriffelement ist mit einer Verdrehsicherung für das feststehende Wälzlagerteil versehen, die als mehreckige, im Wesentlichen kreis- oder kreisbogenförmige, offene, metallische Verdrehsicherung ausgebildet ist.

Überraschender Weise gewährleistet eine derartige metallische Verdrehsicherung eine ausreichende Vorspannung des Wälzlagers, eine zuverlässige Unterbindung der Rotation des feststehenden Wälzlagerteils relativ zum Lagersitz und eine ausreichende Vibrationsdämpfung ohne merkliche Zunahme der Lärmemission. Aufgrund der metallischen Ausbildung, zum Beispiel aus Stahl, insbesondere aus nicht rostendem Stahl, besonders bevorzugt aus Federstahl, oder aus einer Beryllium-Kupfer-Legierung weist die Verdrehsicherung zusätzlich eine im Wesentlichen uneingeschränkte Beständigkeit gegenüber den bei der Reinigung oder der Sterilisation herrschenden Umgebungsbedingungen oder dafür verwendeten Substanzen auf.

Erfindungsgemäß umfasst der Begriff Handgriffelement gerade Handgriffe oder Handstücke, gebogene Handgriffe oder Handstücke, die im Dentalbereich oftmals als Winkelstücke bezeichnet werden, als auch Teile von Handgriffen. Derartige Teile werden zum Beispiel durch einen Griffabschnitt eines Handgriffs gebildet, der insbesondere mit verschiedenen Kopfabschnitten für unterschiedliche Werkzeuge oder mit Getrieben für unterschiedliche Über- oder Untersetzungen verbindbar ist. Die Teile von Handgriffen können jedoch auch als Zwischenstücke, Kupplungen, Adapter oder eigenständige Hülsenabschnitte ausgebildet sein.

Das durch das zumindest eine Wälzlager drehbar gelagerte Drehteil umfasst sowohl Drehteile, die für eine vollständige Drehung, d.h. für eine Drehung von 360° oder mehr, ausgebildet sind, als auch schwenkbare oder oszillierende Drehteile, die Drehungen mit einem Drehwinkel von weniger als 360° ausführen. Das Drehteil weist zum Beispiel eine Welle oder einen oder mehrere Wellenteile, eine Achse, einen Schaft, eine Getriebe oder Getriebeteile, eine Hohlwelle oder bevorzugt eine Werkzeugaufnahmevorrichtung am Vorderende des Handgriffelements zur Aufnahme eines Behandlungswerkzeugs, zum Beispiel eines rotierbaren Bohrers, auf. Die Werkzeugaufnahmevorrichtung umfasst in einem besonders bevorzugten Ausführungsbeispiel eine Hohlwelle, an der ein fluidbetriebenes Laufrad oder ein Antriebszahnrad befestigt ist, und eine in der Hohlwelle aufgenommene Spannzange zum lösbaren Anschluss des Behandlungswerkzeugs.

Die zu Lagerung des Drehteils dienenden Wälzlager sind als Axiallager oder als Radiallager ausgebildet. Die in den Laufringen laufenden Wälzkörper der Wälzlager umfassen zum Beispiel Kugeln, Rollen, Nadeln, Tonnen oder Kegel. In einem Ausführungsbeispiel sind die Wälzlager oder Teile davon aus Metall, insbesondere Stahl, oder aus Keramik hergestellt. In einem anderen Ausführungsbeispiel weisen einzelne Bauteile Beschichtungen auf, zum Beispiel Beschichtungen mit keramischen Werkstoffen, bevorzugt Siliziumnitrid, Zirkoniumnitrid oder Siliziumcarbid, oder mit einer metallhaltigen, harten Kohlenstoffschicht. Unter dem Begriff metallhaltige, harte Kohlenstoffschichten werden alle amorphen Kohlenstoffschichten mit Wasserstoff- bzw. Metalldotierungen (a-C:H; Me-C:H) subsummiert. Als Dotierstoffe werden bevorzugt Tantal, Wolfram, Titan, Chrom, Niob und Legierungen dieser Metalle verwendet. Die Herstellung dieser Kohlenstoffschichten erfolgt bevorzugt durch physikalisches und chemisches Aufdampfen (physical / chemical vapour deposition) auf die metallenen Bauteile.

In einem weiteren Ausführungsbeispiel sind das Wälzlager oder Teile davon mit Schmierstoffen oder Schmiermitteln versehen, die oberflächlich aufgetragen oder in Poren oder Reservoirs aufgenommen sind. In einem Ausführungsbeispiel sind die Wälzkörper in einem Käfig aufgenommen. In einem anderen Ausführungsbeispiel sind an einem Laufring des Wälzlagers Vertiefungen oder Aufnahmen für drehfeste Bauteile des Handgriffelements, insbesondere für die Verdrehsicherung, oder Befestigungs- oder Lagerelemente zur Fixierung des Wälzlagers im Handgriffelement vorgesehen.

Die Verdrehsicherung ist als mehreckiges, bevorzugt als vier- bis zehneckiges, insbesondere als achteckiges Metallelement ausgebildet. Die durch die Ecken eingeschlossenen Winkel sind bevorzugt in etwa gleich groß, wobei die Winkel derart ausgebildet sind, dass die Verdrehsicherung eine kreis- oder kreisbogenförmige Gestalt aufweist. Bevorzugt liegt der durch die Ecken eingeschlossene Winkel in etwa zwischen 30° und 60°. Die Verdrehsicherung weist zwei freie, bevorzugt einander zugewandte, Enden auf, so dass die Verdrehsicherung einen elastischen, flexiblen Federring bildet.

In einem Ausführungsbeispiel weist das Handgriffelement einen Lagersitz auf, wobei die Verdrehsicherung erste, das feststehende Wälzlagerteil kontaktierende Abschnitte und zweite, den Lagersitz kontaktierende Abschnitte aufweist und wobei die beiden Abschnitte abwechselnd auf der Verdrehsicherung angeordnet sind. Durch diese Ausbildung wird in vorteilhafter Weise eine gleichmäßige Übertragung und Verteilung der Kräfte auf die Verdrehsicherung und auf den Lagersitz und eine gleichmäßige Belastung der Verdrehsicherung erzielt.

Der Lagersitz weist eine oder mehrere, winkelig zueinander angeordnete Seitenwände oder Lagerflächen auf und ist insbesondere als Vorsprung, Rücksprung, Schulter, bevorzugt als Ringschulter, Einstich oder Aufnahme ausgebildet. Er umfasst zumindest ein drehfest im Handgriffelement angeordnetes Bauteil, zum Beispiel zumindest einen Teil der Außenhülse des Handgriffelements, einer Wand eines in das Handgriffelement einsetzbaren Patrone oder einer Lagerhülse.

In einem Ausführungsbeispiel sind die zweiten, den Lagersitz kontaktierenden Abschnitte vom Wälzlager beabstandet. Es wird dadurch eine elastische Biegbarkeit der Verdrehsicherung erreicht bzw. die Elastizität der Verdrehsicherung erhöht, wodurch in vorteilhafter Weise Vibrationen des Drehteils während der Rotation abgefangen oder gedämpft werden.

In einem Ausführungsbeispiel weist die Verdrehsicherung mehrere Ecken oder Vorsprünge auf, wobei die Ecken oder Vorsprünge die zweiten, den Lagersitz kontaktierenden Abschnitte bilden. Diese Ausformung der Verdrehsicherung ermöglicht eine besonders feste und sichere Fixierung der Verdrehsicherung und des feststehenden Wälzlagerteils im Handgriffelement.

In einem Ausführungsbeispiel umfasst die Verdrehsicherung mehrere im Wesentlichen gerade, insbesondere stabförmige, Segmente, wobei diese Segmente die ersten, das feststehende Wälzlagerteil kontaktierenden Abschnitte bilden. Es wird damit ein äußerst einfacher und kostengünstiger Aufbau der Verdrehsicherung erreicht, da die einzelnen Segmente gewinkelt zueinander angeordnet sind, so dass an ihren Kontaktpunkten die Ecken oder Vorsprünge entstehen, die die zweiten, den Lagersitz kontaktierenden Abschnitte bilden.

Bevorzugt sind die geraden Segmente derart ausgebildet, dass sie zumindest teilweise an das Wälzlager anschmiegbar sind, wobei sie sich dabei etwas in Richtung des Lagersitzes durchbiegen. Die geraden Segmente berühren das Wälzlager bzw. den feststehende Wälzlagerteil damit nicht nur an einem Punkt sondern über einen größeren Bereich, wodurch eine verbesserte, zuverlässigere Fixierung des feststehenden Wälzlagerteils durch die Verdrehsicherung erzielt wird.

Die Anschmiegbarkeit der Segmente wird insbesondere von deren Abmessungen, insbesondere vom Verhältnis des Durchmessers zur Länge der Segmente und der Elastizität des Materials, aus dem die Verdrehsicherung hergestellt ist, bestimmt. Der Durchmesser der Segmente beträgt bevorzugt etwa 0,15 - 0,4 mm, besonders bevorzugt etwa 0,2 mm. Die Länge der Segmente beträgt bevorzugt etwa 1,5 - 3,0 mm, besonders bevorzugt etwa 2,1 mm. In einer anderen bevorzugten Ausführungsform ist die Verdrehsicherung aus legiertem oder unlegiertem Federstahl, insbesondere aus Federstahl mit der Stahlnummer 1.4310, hergestellt. Zur Legierung werden unter anderem Elemente wie Silizium, Mangan oder Molybdän verwendet. In einer weiteren bevorzugten Ausführungsform weist das Material, aus dem die Verdrehsicherung hergestellt ist, ein E-Modul von etwa 185 - 215 kN/mm² bei 20°C, besonders bevorzugt von etwa 200 kN/mm² bei 20°C auf.

In einem Ausführungsbeispiel weist der Lagersitz eine oder mehrere Aufnahmen, insbesondere eine Nut oder Ringnut, auf, in die die zweiten, den Lagersitz kontaktierenden Abschnitte eingreifen. Damit wird in vorteilhafter Weise eine besonders feste und sichere Fixierung der Verdrehsicherung und des feststehenden Wälzlagerteils im Handgriffelement erreicht.

Um eine zusätzliche Vibrationsdämpfung und Verringerung der Lärmemission zu erreichen ist in einem Ausführungsbeispiel die metallische Verdrehsicherung mit einer Beschichtung, insbesondere mit einer Kunststoffbeschichtung, zum Beispiel mit einer PVC-Schicht oder mit einem Mantel aus Elastomer, umgeben.

Das Verfahren zur Herstellung eines medizinischen, insbesondere dentalen, Handgriffelements beinhaltet folgende Schritte:
- zur Verfügung stellen eines Handgriffelements und eines drehbar im Handgriffelement anordenbaren Drehteils,
- zur Verfügung stellen zumindest eines Wälzlagers zur Lagerung des drehbaren angeordneten Drehteils, wobei das Wälzlager zumindest einen mit dem Drehteil mitrotierbaren Wälzlagerteil und zumindest einen relativ zum Drehteil feststehenden Wälzlagerteil aufweist,
- zur Verfügung stellen einer Verdrehsicherung, die als mehreckiger, im Wesentlichen kreis-oder kreisbogenförmiger, metallischer Federring mit zwei freien Enden, zwischen denen eine Öffnung vorgesehen ist, ausgebildet ist, und
- Einbau des Wälzlagers zur Lagerung des Drehteils im Handgriffelement mit der Verdrehsicherung.

Die Erfindung wird nachfolgend anhand bevorzugter Ausführungsbeispiele und Bezug nehmend auf die beigefügten Zeichnungen erläutert:
Figur 1 zeigt einen Teil eines medizinischen Handgriffelements mit einem durch ein Wälzlager drehbar gelagerten Drehteil, wobei der äußere Laufring des Wälzlagers durch eine mehreckige, im Wesentlichen kreis- oder kreisbogenförmig ausgebildete, offene, metallische Verdrehsicherung im Handgriffelement fixiert ist.
Figur 2 zeigt ein Ausführungsbeispiel einer mehreckigen, im Wesentlichen kreis- oder kreisbogenförmig ausgebildeten, offenen, metallischen Verdrehsicherung.

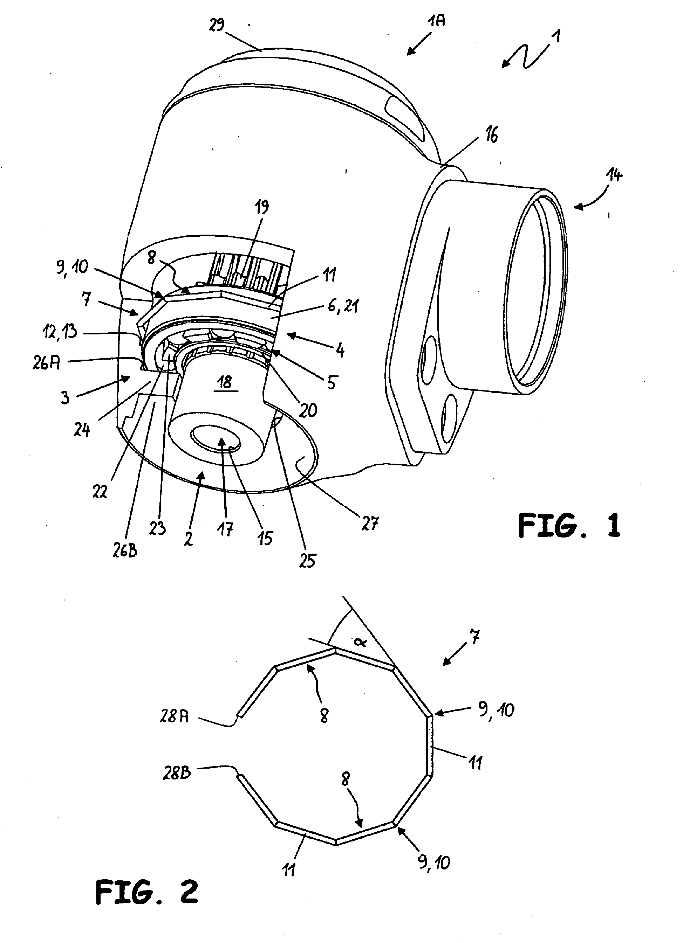

Der in Figur 1 dargestellte Winkelstückkopf 1A ist Teil eines medizinischen, insbesondere dentalmedizinischen, Handgriffelements 1. An einem Ende des Winkelstückkopfs 1A ist eine Anschlussvorrichtung 14 zum Anschluss eines weiteren Teils des Handgriffelements 1, zum Beispiel eines hülsenförmigen Hals- oder Griffabschnittes, vorgesehen. In der Anschlussvorrichtung 14 sind mehrere Bohrungen zur Übertragung von Medien, wie zum Beispiel von Druckluft oder Wasser, oder zur Durchführung von Bauteilen wie Medienleitungen, elektrischen Leitungen oder einer Antriebswelle vorgesehen. Das Handgriffelement 1 ist als so genanntes Winkelstück ausgebildet, bei dem der Griffabschnitt aus zwei zueinander gewinkelt angeordneten Abschnitten besteht und bei dem die Werkzeugöffnung 15 seitlich am Winkelstückkopf 1A angeordnet ist, so dass ein Behandlungswerkzeug quer zum Griffabschnitt in den Winkelstückkopf 1A einsetzbar ist. Eine ein- oder mehrteilige Außenhülse 16 umgibt den Winkelstückkopf 1A. Selbstverständlich kann das Handgriffelement auch andere Außenformen, insbesondere eine gerade oder eine pistolenförmige Gestalt aufweisen.

Im Winkelstückkopf 1A des Handgriffelements 1 befindet sich in bekannter Weise eine über die Werkzeugöffnung 15 zugängliche Werkzeugaufnahme 17. Die Werkzeugaufnahme 17 ist zumindest teilweise in einer Hohlwelle 18 aufgenommen, die beweglich gelagert ist, insbesondere durch zwei Wälzlager, wovon nur ein Wälzlager 4 zu erkennen ist. Die Werkzeugaufnahme 17 umfasst bevorzugt eine Spannzange zur lösbaren Aufnahme eines Behandlungswerkzeugs, zum Beispiel eines rotierbaren Bohrers. Auf der Hohlwelle 18 ist ein Ritzel oder Zahnrad 19 angeordnet, das mit einem weiteren Zahnrad kämmt. Dieses weitere Zahnrad ist mit einer Antriebswelle verbunden, die eine Antriebsbewegung von einer Antriebseinheit, zum Beispiel von einem Luft- oder Elektromotor, überträgt. Alternativ kann an der Hohlwelle 18 ein fluidbetriebenes, bevorzugt mit Druckluft betriebenes, Laufrad befestigt sein, das gemeinsam mit der Hohlwelle 18 und der Werkzeugaufnahme 17 in eine Drehbewegung versetzbar ist.

Auf der der Werkzeugöffnung 15 entgegen gesetzten Seite des Winkelstückkopfs 1A ist eine Werkzeuglösevorrichtung mit einem Druckknopf 29 zur Entnahme eines in der Werkzeugaufnahme 17 eingespannten Werkzeugs vorgesehen.

Durch die Übertragung der Antriebsbewegung von der Antriebseinheit über die Antriebswelle werden das Zahnrad 19, die Hohlwelle 18 und die Werkzeugaufnahme 17 in eine Drehbewegung versetzt. Die Bauteile 17, 18 und 19 bilden somit ein drehbar im Handgriffelement 1 angeordnetes Drehteil 2, das durch das Wälzlager 4 und ein weiteres Wälzlager rotierbar im Winkelstückkopf 1A aufgenommen ist. Da die beiden Wälzlager gleich aufgebaut sind, wird im Weiteren nur das Wälzlager 4 beschrieben, wobei die Beschreibung auf beide Wälzlager zutrifft.

Das als Kugellager ausgebildete Wälzlager 4 ist in einem Lagersitz 3 angeordnet. Der Lagersitz 3 umfasst einen Teil der Außenhülse 16 des Winkelstückkopfs 1A und eine Ringschulter 24, die einstückig mit der Außenhülse 16 ausgebildet ist und die in etwa in einem Winkel von 90° von der Innenwand der Außenhülse 16 absteht. Die Ringschulter 24 ist mit einer Bohrung 25 versehen, durch die ein Abschnitt des Drehteils 2, insbesondere ein Teil der Hohlwelle 18 und der Werkzeugaufnahme 17, ragt. Die Ringschulter 24 weist zwei einander gegenüberliegende Flächen 26A, 26B auf, wobei die dem Zahnrad 19 zugewandte Fläche 26A als Auflagefläche für das Wälzlager 4 dient. Bevorzugt ist zwischen der Fläche 26A und dem Wälzlager 4 noch eine Federscheibe zur axialen Vorspannung des Wälzlagers 4 vorgesehen. Die der Werkzeugöffnung 15 zugewandte Fläche 26B bildet gemeinsam mit einem sich verjüngenden Abschnitt der Außenhülse 16 eine Aufnahme 27 für eine Medienabgabevorrichtung, zum Beispiel für eine Sprayplatte oder für Düsenöffnungen.

Das Wälzlager 4 umfasst einen inneren Laufring 20, einen äußeren Laufring 21 und dazwischen angeordnete Rollelemente in Form von Kugeln 22, die durch einen Wälzlagerkäfig 23 voneinander getrennt sind. Das Wälzlager 4 ist mit dem Drehteil 2 verbunden, zum Beispiel aufgeschoben oder aufgepresst. Der innere Laufring 20 kontaktiert dabei das Drehteil 2 und rotiert ebenso wie die Kugeln 22 und der Käfig 23 mit dem Drehteil 2, so dass die Bauteile 20, 22 und 23 den relativ zum Drehteil 2 und relativ zum Lagersitz 3 rotierbaren Wälzlagerteil 5 bilden. Im Gegensatz dazu steht der äußere Laufring 21 still, so dass er das relativ zum Drehteil 2 und relativ zum Lagersitz 3 feststehende Wälzlagerteil 6 bildet.

Um das feststehende Wälzlagerteil 6 drehfest im Lagersitz 3 und im Handgriffelement 1 anzuordnen, ist eine Verdrehsicherung vorgesehen, die als mehreckige, im Wesentlichen kreisförmige, offene, metallische Verdrehsicherung 7 ausgebildet ist. Die Verdrehsicherung 7 umfasst mehrere, in der Ausführungsform gemäß Figur 2 neun, gerade, insbesondere stabförmige, Segmente 11. Die Segmente 11 weisen einen runden, insbesondere einen kreisrunden, Querschnitt auf, dessen Durchmesser in der Ausführungsform gemäß Figur 2 etwa 0,2 mm beträgt. Ein Einzelsegment 11 der in Figur 2 dargestellten Verdrehsicherung 7 ist in etwa 2,1 mm lang, der Abstand zwischen zwei gegenüber liegenden Segmenten 11 beträgt etwa 6,5 mm.

Die Segmente 11 sind gewinkelt zueinander angeordnet sind, so dass an den Kontaktstellen zwischen zwei Segmenten 11 je eine Ecke 10, in der Ausführungsform gemäß Figur 2 somit insgesamt acht Ecken 10, ausgebildet sind. Der Winkel α zwischen zwei Segmenten 11 der in der Figur 2 dargestellten Verdrehsicherung 7 beträgt etwa 36°. Die Verdrehsicherung 7 hat eine im Wesentlichen kreisförmige Form, sie weist jedoch zwei freie Enden 28A, 28B auf, zwischen denen eine Öffnung vorgesehen ist, so dass die Verdrehsicherung 7 als offener Federring ausgebildet ist. Der Abstand zwischen den beiden freien Enden 28A, 28B entspricht bei der in der Figur 2 dargestellten Verdrehsicherung 7 in etwa der Länge eines Segments 11, er kann jedoch selbstverständlich auch größer oder geringer sein.

Wie aus der Figur 1 zu erkennen ist, ist am Lagersitz 3 eine Aufnahme 12 in Form einer sich entlang der gesamten Innenwand der Außenhülle 16 erstreckenden Ringnut 13 vorgesehen, in die Teile der Verdrehsicherung 7 eingreifen. Insbesondere ragen die Ecken 10 in die Ringnut 13 und bilden damit die zweiten, den Lagersitz 3 kontaktierenden Abschnitte 9, wohingegen Teile der Segmente 11 das Wälzlager 4 berühren und nicht in die Ringnut 13 ragen und somit die ersten, das Wälzlager 4 kontaktierenden Abschnitte 8 der Verdrehsicherung 7 bilden. Die Verdrehsicherung 7 umgibt das Wälzlager 4 in etwa in halber Höhe des Wälzlagers 4. Entsprechend ist auch die Aufnahme 12 in etwa eine halbe Wälzlagerhöhe von der Ringschulterfläche 16 entfernt.

Die Erfindung ist nicht auf das beschriebene Ausführungsbeispiel beschränkt, sondern umfasst alle Ausführungen, die das prinzipielle, sinngemäße Funktionsprinzip der Erfindung anwenden oder beinhalten. So kann die Verdrehsicherung insbesondere auch aus mehreren, zum Beispiel zwei oder drei, Einzelkomponenten bestehen, die jeweils eine kreisbogenförmige Form aufweisen und die derart zusammenfügbar oder im Handgriffelement verbaubar sind, dass sie eine im Wesentlichen kreisförmige Verdrehsicherung ergeben.

## Patentansprüche

1. Medizinisches, insbesondere dentales, Handgriffelement (1) mit
einem drehbar angeordneten Drehteil (2), zumindest einem Wälzlager (4) zur Lagerung des drehbar angeordneten Drehteils (2), wobei das Wälzlager (4) zumindest einen mit dem Drehteil (2) mitrotierbaren Wälzlagerteil (5) und zumindest einen relativ zum Drehteil (2) feststehenden Wälzlagerteil (6) aufweist, und mit einer Verdrehsicherung (7) für das feststehende Wälzlagerteil (6), **dadurch gekennzeichnet, dass**
die Verdrehsicherung (7) als mehreckiger, im Wesentlichen kreis- oder kreisbogenförmiger, metallischer Federring mit zwei freien Enden (28A, 28B), zwischen denen eine Öffnung vorgesehen ist, ausgebildet ist.

2. Handgriffelement (1) nach Anspruch 1, **gekennzeichnet durch**
einen Lagersitz (3), wobei die Verdrehsicherung (7) erste, das feststehende Wälzlagerteil (6) kontaktierende Abschnitte (8) und zweite, den Lagersitz (3) kontaktierende Abschnitte (9) aufweist.

3. Handgriffelement (1) nach Anspruch 2, **dadurch gekennzeichnet, dass**
die ersten das feststehende Wälzlagerteil (6) kontaktierenden Abschnitte (8) und die zweiten, den Lagersitz (3) kontaktierenden Abschnitte (9) abwechselnd auf der Verdrehsicherung (7) angeordnet sind.

4. Handgriffelement (1) nach einem der Ansprüche 2 oder 3, **dadurch gekennzeichnet, dass**
die zweiten, den Lagersitz (3) kontaktierenden Abschnitte (9) vom Wälzlager (4) beabstandet sind.

5. Handgriffelement (1) nach einem der Ansprüche 2 bis 4, **dadurch gekennzeichnet,**
**dass**
die Verdrehsicherung (7) mehrere Ecken (10) oder Vorsprünge umfasst, wobei die Ecken (10) oder Vorsprünge die zweiten, den Lagersitz (3) kontaktierenden Abschnitte (9) bilden.

6. Handgriffelement (1) nach einem der Ansprüche 2 bis 5, **dadurch gekennzeichnet,**
**dass**
die Verdrehsicherung (7) mehrere im Wesentlichen gerade, insbesondere stabförmige, Segmente (11), umfasst, wobei diese Segmente (11) die ersten, das feststehende Wälzlagerteil (6) kontaktierenden Abschnitte (8) bilden.

7. Handgriffelement (1) nach Anspruch 6, **dadurch gekennzeichnet, dass**
die geraden Segmente (11) derart ausgebildet sind, dass sie zumindest teilsweise an das Wälzlager (4) anschmiegbar sind.

8. Handgriffelement (1) nach einem der Ansprüche 2 bis 7, **dadurch gekennzeichnet, dass**
der Lagersitz (3) eine oder mehrere Aufnahmen (12) zum Eingriff der zweiten, den Lagersitz (3) kontaktierenden Abschnitte (9) aufweist, wobei die zumindest eine Aufnahme (12) insbesondere eine Nut oder eine Ringnut (13) umfasst.

9. Handgriffelement (1) nach einem der vorhergehenden Ansprüche, **dadurch gekennzeichnet, dass**
die Verdrehsicherung (7) aus Stahl, vorzugsweise aus Federstahl, hergestellt ist.

10. Handgriffelement (1) nach einem der vorhergehenden Ansprüche, **dadurch gekennzeichnet, dass**
die Verdrehsicherung (7) mit einer Beschichtung, Insbesondere mit einer Kunststoffbeschichtung, versehen ist.

11. Handgriffelement (1) nach einem der vorhergehenden Ansprüche, **dadurch gekennzeichnet, dass**
das Drehteil (2) eine Werkzeugaufnahme (17) zur Aufnahme eines Behandlungswerkzeugs umfasst.

12. Handgriffelement (1) nach einem der vorhergehenden Ansprüche, **dadurch gekennzeichnet, dass** die Verdrehsicherung (7) mehrere gerade, insbesondere stabförmige Segmente (11) umfasst, die gewinkelt zueinander angeordnet sind, sodass an den Kontaktstellen zwischen zwei Segmenten (11) je eine Ecke (10) ausgebildet ist.

13. Handgriffelement (1) nach einem der vorhergehenden Ansprüche, **dadurch gekennzeichnet, dass** die Verdrehsicherung (7) mehrere gerade, insbesondere stabförmige Segmente (11) umfasst, die gewinkelt zueinander angeordnet sind, wobei die freien Enden (28A, 28B) einander zugewandt sind.

14. Verfahren zur Herstellung eines medizinischen, insbesondere dentalen, Handgriffelements (1) mit den Schritten:
- zur Verfügung stellen eines Handgriffelements (1) und eines drehbar Im Handgriffelement (1) angeordneten Drehteils (2),
- zur Verfügung stellen zumindest eines Wälzlagers (4) zur Lagerung des drehbar angeordneten Drehteils (2), wobei das Wälzlager (4) zumindest einen mit dem Drehteil (2) mitrotierbaren Wälzlagerteil (5) und zumindest einen relativ zum Drehteil (2) feststehenden Wälzlagerteil (6) aufweist,
**gekennzeichnet durch**
- das zur Verfügung stellen einer Verdrehsicherung (7), die als mehreckiger, im Wesentlichen kreis- oder kreisbogenförmiger, metallischer Federring mit zwei freien Enden (28A, 28B), zwischen denen eine Öffnung vorgesehen ist, ausgebildet ist, und **durch**
- den Einbau des Wälzlagers (4) zur Lagerung des Drehteils (2) im Handgriffelement (1) mit der Verdrehsicherung (7).

## Claims

1. Medical, in particular dental, handheld element (1) having
a rotatably arranged rotary part (2), at least one roller bearing (4) for bearing support of the rotatably arranged rotary part (2), wherein the roller bearing (4) has at least one roller bearing part (5) that is co-rotational with the rotary part (2) and at least one roller bearing part (6) that is stationary in relation to the rotary part (2), and an anti-twist device (7) for the stationary roller bearing part (6), **characterized in that**
the anti-twist device (7) is designed as a polygonal, substantially circular or arc-shaped, metallic spring ring with two free ends (28A, 28B), between which an opening is provided.

2. The handheld element (1) according to claim 1, **characterized by**
a bearing seat (3), wherein the anti-twist device (7) has first sections (8) contacting the stationary roller bearing part (6) and second sections (9) contacting the bearing seat (3).

3. The handheld element (1) according to claim 2, **characterized in that**
the first sections (8) contacting the stationary roller bearing part (6) and the second sections (9) contacting the bearing seat (3) are arranged in alternation on the anti-twist device (7).

4. The handheld element (1) according to one of the claims 2 or 3, **characterized in that**
the second sections (9) contacting the bearing seat (3) are arranged at a distance from the roller bearing (4).

5. The handheld element (1) according to one of the claims 2 to 4, **characterized in that**
the anti-twist device (7) comprises multiple corners (10) or protrusions, wherein the corners (10) or protrusions form the second sections (9) contacting the bearing seat (3).

6. The handheld element (1) according to one of the claims 2 to 5, **characterized in that**
the anti-twist device (7) comprises a plurality of substantially straight, in particular rod-shaped segments (11), wherein these segments (11) form the first sections (8) contacting the stationary roller bearing part (6).

7. The handheld element (1) according to Claim 6, **characterized in that**
the straight segments (11) are designed so that they at least partially adapt to the roller bearing (4).

8. The handheld element (1) according to one of the claims 2 to 7, **characterized in that**
the bearing seat (3) has one or more receptacles (12) for engagement of the second sections (9) contacting the bearing seat (3), wherein the at least one receptacle (12) comprises in particular a groove or a ring groove (13).

9. The handheld element (1) according to one of the preceding claims, **characterized in that**
the anti-twist device (7) is made of steel, preferably spring steel.

10. The handheld element (1) according to one of the preceding claims, **characterized in that**
the anti-twist device (7) is provided with a coating, in particular with a plastic coating.

11. The handheld element (1) according to one of the preceding claims, **characterized in that**
the rotary part (2) comprises a tool receptacle (17) for accommodating a treatment tool.

12. The handheld element (1) according to one of the preceding claims, **characterized in that**
the anti-twist device (7) comprises a plurality of straight, in particular rod-shaped segments (11), which are arranged at an angle to one another, so that at the contact points between two segments (11) one corner (10) each is formed.

13. The handheld element (1) according to one of the preceding claims, **characterized in that**
the anti-twist device (7) comprises a plurality of straight, in particular rod-shaped segments (11), which are arranged at an angle to one another, wherein the free ends (28A, 28B) face one another.

14. A method for manufacturing a medical, in particular dental, handheld element (1), comprising the steps:
- providing a handheld element (1) and a rotary part (2) rotatably arrangeable in the handheld element (1),
- providing at least one rolling bearing (4) for bearing support of the rotatably arranged rotary part (2), wherein the roller bearing (4) has at least one roller bearing part (5) that is co-rotational with the rotary part (2) and at least one roller bearing part (6) that is stationary relative to the rotary part (2),
**characterized by**
- providing an anti-twist device (7) which is designed as a polygonal, substantially circular or arc-shaped, metallic spring ring with two free ends (28A, 28B), between which an opening is provided, and
- installing the roller bearing (4) for bearing support of the rotary part (2) in the handheld element (1) with the anti-twist device (7).

## Revendications

1. Elément de préhension manuelle, notamment dentaire (1), comportant
une pièce tournante disposée en rotation (2), au moins un palier à roulement (4) pour l'appui de la pièce tournante disposée en rotation (2), le palier à roulement (4) présentant au moins un élément de palier de roulement (5) pouvant tourner avec la pièce tournante (2) et au moins un élément de palier de roulement (6) fixe par rapport à la pièce tournante (2) et un système anti-torsion (7) pour l'élément fixe de palier de roulement (6), **caractérisé en ce que**
le système anti-torsion (7) est réalisé sous forme d'une rondelle élastique métallique polygonale sensiblement en forme de cercle ou d'arc de cercle et comportant deux extrémités libres (28A, 28B) entre lesquelles une ouverture est pratiquée.

2. Elément de préhension manuelle (1) selon la revendication 1, **caractérisé par**
un siège de palier (3), le système anti-torsion (7) présentant de premières sections (8) en contact avec l'élément de palier fixe (6) et de secondes sections (9) en contact avec le siège de palier (3).

3. Elément de préhension manuelle (1) selon la revendication 2, **caractérisé en ce que**
les premières sections (8) en contact avec l'élément de palier fixe (6) et les secondes sections (9) en contact avec le siège de palier (3) sont disposées en alternance sur le système anti-torsion (7).

4. Elément de préhension manuelle (1) selon une des revendications 2 ou 3, **caractérisé en ce que**
les secondes sections (9) en contact avec le siège de palier (3) sont espacées du palier à roulement (4).

5. Elément de préhension manuelle (1) selon une des revendications 2 à 4, **caractérisé en ce que**
le système anti-torsion (7) comprend plusieurs angles (10) ou saillies, les angles (10) ou saillies formant les secondes sections (9) en contact avec le siège de palier (3).

6. Elément de préhension manuelle (1) selon une des revendications 2 à 5, **caractérisé en ce que**
le système anti-torsion (7) comprend plusieurs segments sensiblement droits, notamment en forme de barres (11), ces segments (11) formant les premières sections (8) en contact avec l'élément fixe de palier (6).

7. Elément de préhension manuelle (1) selon la revendication 6, **caractérisé en ce que**
les segments droits (11) sont réalisés de manière à pouvoir fléchir du moins partiellement vers le palier à roulement (4).

8. Elément de préhension manuelle (1) selon une des revendications 2 à 7, **caractérisé en ce que**
le siège de palier (3) présente un ou plusieurs supports (12) pour l'engrènement des secondes sections (9) en contact avec le siège de palier (3), l'au moins un support (12) comprenant notamment une rainure ou une rainure annulaire (13).

9. Elément de préhension manuelle (1) selon une des revendications précédentes, **caractérisé en ce que**
le système anti-torsion (7) est fabriqué en acier, de préférence en acier à ressort.

10. Elément de préhension manuelle (1) selon une des revendications précédentes, **caractérisé en ce que**
le système anti-torsion (7) est pourvu d'un revêtement, notamment d'un revêtement en matière plastique.

11. Elément de préhension manuelle (1) selon une des revendications précédentes, **caractérisé en ce que**
la pièce tournante (2) comporte un porte-outil (17) destiné à recevoir un outil de traitement.

12. Elément de préhension manuelle (1) selon une des revendications précédentes, **caractérisé en ce que**
le système anti-torsion (7) comprend plusieurs segments droits, notamment en forme de barres (11), qui sont disposés en angle les uns par rapport aux autres, de sorte qu'un angle (10) est chaque fois formé aux points de contact entre deux segments (11).

13. Elément de préhension manuelle (1) selon une des revendications précédentes, **caractérisé en ce que**
le système anti-torsion (7) comprend plusieurs segments droits, notamment en forme de barres (11), qui sont disposés en angle les uns par rapport aux autres, les extrémités libres (28A, 28B) étant tournées les unes vers les autres.

14. Procédé d'un élément de préhension manuelle médical, notamment dentaire (1), comportant les étapes suivantes :
- mise à disposition d'un élément de préhension manuelle (1) et d'une pièce tournante (2) disposée en rotation dans l'élément de préhension manuelle (1),
- mise à disposition d'au moins un palier à roulement (4) pour l'appui de la pièce tournante disposée en rotation (2), le palier à roulement (4) présentant au moins un élément de palier de roulement (5) pouvant tourner avec la pièce tournante (2) et au moins un élément de palier de roulement (6) fixe par rapport à la pièce tournante (2),
**caractérisé par**
- la mise à disposition d'un système anti-torsion (7), réalisé sous forme d'une rondelle élastique métallique polygonale sensiblement en forme de cercle ou d'arc de cercle et comportant deux extrémités libres (28A, 28B) entre lesquelles une ouverture est pratiquée, et par
- l'intégration du palier à roulement (4) pour l'appui de la pièce tournante (2) dans l'élément de préhension manuelle (1) avec le système anti-torsion (7).
